# EUROPÄISCHE PATENTSCHRIFT

(11) **EP 3 407 677 B1**
(45) Veröffentlichungstag und Bekanntmachung des Hinweises auf die Patenterteilung: **06.04.2022**
(21) Anmeldenummer: 18172913.8
(22) Anmeldetag: 17.05.2018
(51) Int. Cl.: H05B 3/34, H05B 1/02, A24F 40/44, A24F 40/46, A24F 40/50

(54) **VERDAMPFEREINHEIT FÜR EINEN INHALATOR UND VERFAHREN ZUM STEUERN EINER VERDAMPFEREINHEIT**
EVAPORATOR UNIT FOR AN INHALER AND METHOD FOR CONTROLLING AN EVAPORATOR UNIT
UNITÉ ÉVAPORATEUR POUR UN INHALATEUR ET PROCÉDÉ DE COMMANDE D'UNE UNITÉ ÉVAPORATEUR

(30) Priorität: 24.05.2017 DE 102017111435
(43) Veröffentlichungstag der Anmeldung: 28.11.2018
(73) Patentinhaber: Hauni Maschinenbau GmbH, 21033 Hamburg (DE)
(72) Erfinder: SCHMIDT, Rene, 21244 Buchholz i.d.N. (DE); KESSLER, Marc, 22415 Hamburg (DE)
(74) Vertreter: Müller Verweyen

(56) Entgegenhaltungen:
- EP-A1- 3 061 358
- EP-A1- 3 078 281
- WO-A1-2015/117700

## Beschreibung

Die vorliegende Erfindung betrifft eine Verdampfereinheit für einen Inhalator mit einem Heizelement, wobei die Verdampfereinheit zum Verdampfen von Flüssigkeit eingerichtet ist, wobei die verdampfte Flüssigkeit von einem durch die Verdampfereinheit strömenden Luftstrom aufgenommen wird. Die Erfindung betrifft des Weiteren ein Verfahren zum Steuern einer Verdampfereinheit.

Herkömmliche E-Zigaretten bestehen aus einem Docht, beispielsweise aus Glasfaser oder Baumwolle, und einem um den Docht gewickelten Heizdraht oder einem auf dem Docht aufliegenden bzw. den Docht umfassenden Heizgitter. Das Liquid wird in den Docht gesaugt und dort durch Anlegen einer Spannung an den Heizdraht oder das Heizgitter verdampft. Diese Methode hat schwerwiegende Nachteile. Erstens variiert die Anordnung von Docht und Heizer fertigungsbedingt sehr. Dies führt dazu, dass je nach Produktionsexemplar eine unterschiedlich Menge Liquid und damit auch Nikotin pro Zug verdampft wird. Zweitens treten sogenannte Hot-Spots am Heizer auf. Bereiche des Heizers, an denen kein Liquid zur Verfügung steht, Schwachstellen im Heizdraht oder Strukturfehler im Heizgitter, oder besonders eng gewickelte Bereiche der Heizdrähte heizen sich stärker auf als die anderen Bereiche des Heizers. In diesen Bereichen steigt der spezifische Widerstand, was die Heizleistung weiter erhöht, sodass insgesamt eine deutlich überhitzte Stelle entsteht. Als Folge können schädliche Zersetzungsprodukte und der sogenannte "Dry Puff" auftreten. Drittens reichert sich durch die Verdampfung der niedrigsiedenden Liquidbestandteile das Liquid in direkter Nähe zum Heizer mit hochsiedenden Komponenten an. Dies kann im Extremfall dazu führen, dass das Liquid nur abseits dieser Schicht siedet und nichtverdampftes hochsiedendes Liquid aus der heizernahen Schicht mitreißt. Dies führt zu einem ungewollten, explosionsartigen Spritzen des Liquids.

Aktuelle auf dem Docht-Wendel-Prinzip aufbauende elektronische Zigaretten, wie beispielhaft in der US 2016/0021930 A1 beschrieben, weisen verschiedene Nachteile auf. Erstens sind Liquidverdampfung und -dosierung nicht voneinander getrennt. Zweitens stehen Dampfmenge und Heizertemperatur in einem direkten Zusammenhang, d.h. hohe Dampfmengen bedingen hohe Verdampfertemperaturen. Drittens kommt es zu ungleichmäßigen Temperaturbereichen am/im Bereich des Verdampfers mit der Gefahr lokaler Liquid-Überhitzung und Schadstoffentstehung. Viertens weicht die im Verdampferbereich an der Docht-/Wendeloberfläche anliegende Temperatur von der Kerntemperatur im Docht ab, wodurch es zu Konzentrationsänderungen der Liquidbestandteile während eines jeden einzelnen Verdampfungsvorgangs bzw. Puffs oder Zugs kommt. Diese Konzentrationsänderung führt darüber hinaus zu einer allmählichen Veränderung der Zusammensetzung des noch im Flüssigkeitsspeicher befindlichen Liquids, d.h. auch die freigesetzte Wirkstoffmenge ist nicht gleichmäßig und ändert sich von Zug zu Zug.

Die Erzeugung eines inhalierbaren Aerosols auf Basis sogenannter E-Liquids ist die Hauptfunktion von E-Zigaretten. Dabei wird das verabreichte E-Liquid verdampft und durch teilweise oder vollständige Rückkondensation bei der Vermischung mit dem mitgezogenen Luftstrom das Aerosol erzeugt. Die Tröpfchengröße hängt dabei von Faktoren ab, unter anderem Kondensationskeimen in der Luft und Abkühlgeschwindigkeit, die nicht oder nur wenig kontrollierbar sind und in der Anwendung zu einer gegenüber herkömmlichen Zigaretten verzögerten Nikotinaufnahme und somit reduziertem Genußempfinden führen können. Eine Möglichkeit, in dem Verdampfungsverfahren die Tröpfchengrößen und -zusammensetzungen beeinflussen zu können, wäre wünschenswert.

Bei der Verdampfung von E-Liquids in herkömmlichen Docht-Wendel-Verdampfern von E-Zigaretten stellt sich im Docht eine mit Glycerin angereicherte Zusammensetzung ein, bei der die Gasphasenzusammensetzung der des nachströmenden Liquids entspricht. Dazu gehört eine entsprechende Siedetemperatur. Im Docht stellt sich eine mit Glycerin angereicherte Zusammensetzung nahe der Heizwendel ein, die entlang des Dochtes nicht konstant ist. Es lässt sich zeigen, dass in diesem Fall in Teilbereichen des heizerumfassten Dochtes höhere Siedetemperaturen vorkommen müssen, als wenn das Liquid im Bereich der Dampfbildung eine konstante Zusammensetzung hat, d.h. wenn es gut durchmischt ist. Des Weiteren kann ein Zusammensetzungsgradient im Dochtquerschnitt zu einem schlecht zu kontrollierenden spontanen Verdampfen von Liquid in der Dochtmitte führen, was zur Freisetzung von großen Liquidtropfen führt. Im Sinne einer möglichst gleichmäßigen Verdampfung bei möglichst geringer Temperatur zur Vermeidung von thermischer Zersetzung des Liquids sollte dieser Zustand vermieden werden.

Auch andere Verfahren zur Verdampfung, bei der die Wärme durch Heizflächen bzw. feste Wände in das Liquid eingebracht wird, verursachen Blasen- oder Filmsieden und damit lokal stark nichtstationäre Verdampfungsvorgänge mit der Gefahr der spontanen Freisetzung von großen Liquidtropfen und lokaler Überhitzung, da sich Wärmezufuhr und Bildung der Gasphase entgegenstehen.

Aus US2016/0007653 A ist eine Verdampferstruktur in MEMS-Technologie bekannt. Hier befindet sich der Heizer auf einer geschlossenen Membran, sodass der Austrittsort des Dampfes und der Ort der Erhitzung getrennt sind. Daher kann auch hier das oben beschriebene explosionsartige Spritzen des Liquids auftreten. Zudem werden hier ein Heizer und ein Temperatursensor an separaten Stellen auf dieser Membran angebracht, sodass die lokale Heizertemperatur direkt nicht gemessen werden kann. Hinsichtlich der Betriebsart werden keine Angaben gemacht, sodass von einem kontinuierlichen, nicht-gepulsten Betrieb ausgegangen werden muss. Daher besteht die Gefahr einer vollständigen Verdampfung am Heizer mit einem nachfolgenden starken Temperaturanstieg. Als Vorteile der Anmeldung werden die günstige Herstellung und Montage, sowie die Kopplung mit Temperatursensoren und Strömungssensoren angegeben. Die oben beschriebenen Nachteile der herkömmlichen E-Zigarette können damit jedoch nicht eindeutig behoben werden.

Das Heizelement herkömmlicher E-Zigaretten besteht beispielsweise aus einem Chrom-Nickel-Draht, Edelstahl, oder anderen metallischen Legierungen. Diese Legierungen haben den Nachteil, dass ihr spezifischer Widerstand mit der Temperatur ansteigt, sodass schlecht gekühlte Bereiche einen erhöhten Widerstand aufweisen und somit einer erhöhten Heizleistung bedürfen. Dieser selbstverstärkende Prozess führt zu stark inhomogenen Temperaturverteilungen, welche wiederum eine erhöhte und unkontrollierte Schadstofffreisetzung verursachen. Zudem kann die katalytische Wirkung der Metalle zu einer erhöhten Erzeugung von Schadstoffen führen.

Die EP 3 061 358 A1 offenbart eine Verdampfereinheit für eine elektronische Rauchvorrichtung gemäß dem Oberbegriff von Anspruch 1.

WO 2015 117700 A1 und EP 3 078 281 A1 offenbaren weitere Verdampfereinheiten für elektronische Zigaretten.

Die Aufgabe der Erfindung besteht darin, eine einfache, zuverlässig und reproduzierbar arbeitende Verdampfereinheit bereitzustellen, mit deren Hilfe Liquid zuverlässig und reproduzierbar in die Gasphase überführt werden kann.

Die Erfindung löst diese Aufgabe mit den Merkmalen der unabhängigen Ansprüche. Erfindungsgemäß ist das Heizelement zum Erwärmen des Luftstroms eingerichtet und der Verdampfer ist stromabwärts von dem Heizelement angeordnet, und der Verdampfer weist mindestens eine luftdurchlässige, kapillare Struktur mit einer Oberfläche auf, die mindestens teilweise mit Flüssigkeit aus dem Flüssigkeitsspeicher benetzbar ist und durch Kapillarwirkung mit Flüssigkeit versorgt wird. Aufgrund des automatischen Nachströmens von Flüssigkeit infolge der Kapillarwirkung der kapillaren Struktur zu Ausgleich von verdampfter Flüssigkeit ist ein separater aktiver Fördermechanismus entbehrlich, was zu einem verringerten Aufwand führt und den erforderlichen Bauraum verringert.

Durch den Verdampfer mit der luftdurchlässigen, kapillaren Struktur, welche stromabwärts von dem Heizelement angeordnet ist, wird eine äußerst präzise zeitliche Steuerung der Verdampfung und Dosierung der Heizleistung sowie des Dampfes ermöglicht.

In einer bevorzugten Ausführungsform besteht die kapillare Struktur aus einem elektrisch leitfähigen Material, zumindest teilweise. In dieser Ausführungsform kann die kapillare Struktur elektrischen Strom leiten und somit Energie und/oder Signale transportieren.

Vorzugsweise ist die kapillare Struktur beheizbar. Eine beheizbare kapillare Struktur erlaubt die Aufteilung der Heizleistung, die zum Verdampfen der Flüssigkeit erforderlich ist, zwischen dem Heizelement und der kapillaren Struktur. Die Beheizung der kapillaren Struktur bewirkt vorzugsweise eine Grunderwärmung, welche in etwa die erforderliche Leistung zur Erwärmung der nachströmenden Flüssigkeit auf eine Temperatur nahe, vorzugsweise unterhalb, einer Siedetemperatur entspricht. Unterhalb einer Siedetemperatur ist die Flüssigkeit nicht verdampft und kann durch Kapillarwirkung in der kapillaren Struktur und/oder auf der Oberfläche der kapillaren Struktur transportiert werden. Das Heizelement kann die Luft auf eine eingestellte Temperatur, vorzugsweise nahe einer Siedetemperatur der Flüssigkeit, besonders bevorzugt oberhalb einer Siedetemperatur, erhitzen und auf der Oberfläche der kapillaren Struktur befindlichen Flüssigkeit verdampfen. Mit anderen Worten erfolgt die geregelte Vorwärmung des Liquids vorzugsweise bis knapp unter die Siedetemperatur in der kapillaren Struktur. Die von dem Heizelement erzeugte erwärmte Luft liefert dann im Wesentlichen nur noch die Differenz der Verdampfungsenthalpie, die erforderlich ist, um einen Wärmeeintrag in das Liquid zum Überschreiten der Verdampfungsschwelle zu erreichen.

Diese Ausführungsform ist besonders bevorzugt, wenn die kapillare Struktur zudem elektrisch leitend ist. Ist die kapillare Struktur elektrisch leitend, kann der der kapillaren Struktur innewohnende elektrische Widerstand genutzt werden, um die elektrische Energie in thermische Energie umzuwandeln und so die kapillare Struktur zu erhitzen um die Verdampfung der Flüssigkeit auf der Oberfläche der kapillaren Struktur zu erzielen. Die möglichen Wärmestromdichten an der Oberfläche sind zwar kleiner als beim Blasensieden, dies wird durch eine entsprechend große Oberfläche der räumlich strukturierten kapillaren Struktur kompensiert. Damit ist sichergestellt, dass in Kombination mit der großen Oberfläche der kapillaren Struktur und dessen integrierter Heizung eine Verdampfung der Flüssigkeit in gewünschter Menge erfolgen kann, ohne dass Überhitzung oder Blasensieden in unerwünschtem Maße auftritt.

Es ist von Vorteil, den Luftstrom auf eine Temperatur oberhalb einer Siedetemperatur des Flüssigkeitsgemisches zu erhitzen und/oder die kapillare Struktur auf eine Temperatur unterhalb einer Siedetemperatur des Flüssigkeitsgemisches und/oder auf eine Temperatur von mindestens 100 °C, vorzugsweise mindestens 150 °C, weiter vorzugsweise mindestens 200 °C und noch weiter vorzugsweise mindestens 250 °C zu beheizen. Ein Aufheizen der Temperatur im Luftstrom oberhalb einer Siedetemperatur und/oder der kapillaren Struktur auf eine Temperatur oberhalb einer Siedetemperatur und/oder oberhalb einer der angegebenen Werte dient dem blasenfreien Verdampfen in der kapillaren Struktur und der zuverlässigen Liquidförderung in/auf der kapillaren Struktur.

Es ist von Vorteil, dass die kapillare Struktur mittels einer ersten elektrischen/elektronischen Einheit beheizbar ist, wobei die erste elektrische/elektronische Einheit eingerichtet ist, das Heizelement zu steuern und/oder zu regeln. Diese Ausführungsform kommt mit nur einer Leistungselektronik aus, die das Heizelement sowie die kapillare Struktur gemeinsam mit elektrischer Leistung versorgt. Vorzugsweise erfolgt die Leistungsaufteilung durch Einstellung des Verhältnisses der elektrischen Widerstände des Heizelements und der kapillaren Struktur. Eine temperaturabhängige Leistungsaufnahme ist durch die geeignete Wahl von Thermistoren, d.h. Heizmaterialien mit unterschiedlichen Widerstands-Temperaturverhalten, möglich. Die Erfassung der Widerstände des Heizelements und der kapillaren Struktur kann durch eine Messung des jeweiligen Spannungsabfalls erfolgen. Typischerweise wird die Leistungselektronik durch Pulsweitenmodulation geregelt und/oder gesteuert betrieben und durch eine Stromquelle mit Energie versorgt.

Es ist vorteilhaft, dass eine erste elektrische/elektronische Einheit eingerichtet ist, das Heizelement zu steuern und/oder zu regeln, und die kapillare Struktur mittels einer zweiten elektrischen/elektronischen Einheit beheizbar ist. Das Heizelement wird durch eine Leistungselektronik typischerweise durch Pulsweitenmodulation geregelt oder gesteuert betrieben, welche wiederum durch eine Stromquelle mit Energie versorgt wird. Die Beheizung der kapillaren Struktur erfolgt idealerweise durch elektrische Eigenerwärmung, d. h. den ohmschen Widerstand, gesteuert oder geregelt durch eine zweite Leistungselektronik. Auch das Vorhandensein zweier separater Stromquellen für je die erste und zweite elektrische/elektronische Einheit ist denkbar.

Die kapillare Struktur weist vorteilhaft in Durchströmungsrichtung des Luftstroms und/oder quer zu ihrer vorzugsweise flächigen Erstreckung eine Porosität zwischen 5-95%, bevorzugt 10-90%, 15-85%, 20-80%, besonders bevorzugt 30-70%, 35-65% auf. Unter Porosität ist dabei das Verhältnis von Freifläche zu Materialfläche zu verstehen, etwa im Sinne eines Lochflächenanteils der kapillaren Struktur zum Durchtritt des Luftstroms.

Vorzugsweise ist die kapillare Struktur vlies- oder gewebeartig, insbesondere in Form eines gefalteten oder gerollten Metallgewebes. Diese Ausführungsform trägt einer idealerweise durch räumliche Strukturierung möglichst großen, von Liquid benetzbaren inneren Oberfläche Rechnung, womit ein möglichst effektiver und gezielter Wärmeübergang zwischen der kapillaren Struktur und dem Liquid erzielt wird. Diese Ausführungsformen eignen sich zum Transport von Flüssigkeit innerhalb und/oder auf der Oberfläche der kapillaren Struktur von Flüssigkeit durch Kapillarwirkung.

Bevorzugt ist die kapillare Struktur eine mikromechanisch hergestellte Struktur (mikro-elektromechanische Struktur oder MEMS-Struktur) mit einem Substrat und in dem Substrat verlaufenden Kanälen und/oder Rippen. Diese Ausführungsform führt zu einer besonders großen Oberfläche der kapillaren Struktur, was ein gezieltes Verdampfen ermöglicht. Das gezielte oder ungezielte Einbringen von Kanälen und/oder Rippen ermöglicht den Transport von Flüssigkeit innerhalb und/oder auf der Oberfläche der kapillaren Struktur durch Kapillarwirkung.

Vorzugsweise lehnen sich Form und Geometrie der Strukturelemente der kapillaren Struktur und/oder der Heizstrukturen des Heizelements an bionische Strukturen, ähnlich wie beispielsweise Mikrofibrillen, an. Bionische Strukturen weisen eine besonders große Oberfläche aus, welche hier gezielt für das Erwärmen von Luft und/oder dem Bereitstellen von zu verdampfender Flüssigkeit verwendet werden kann. Auch die Herstellung der bionischen Strukturen kann durch geeignete selbstorganisierte Prozesse besonders effizient und kostengünstig erfolgen.

Vorzugsweise sind Betriebsbedingungen der kapillaren Struktur mit einem Sensor messbar. Diese Ausführungsform erlaubt vorzugsweise mittels eines Temperatursensors eine Temperaturüberwachung und kann dabei durch die Erfassung des elektrischen Widerstands des Kapillarverdampfers erfolgen, sofern dieser einen geeigneten Temperaturkoeffizienten aufweist, oder durch einen optionalen dedizierten Temperaturfühler. Vorzugsweise können dafür Heiß- und/oder Kaltleiter (NTC/PTC) verwendet werden. Vorzugsweise können auch der Druck und/oder Strömungsgeschwindigkeiten und/oder Konzentrationen gemessen werden, welche den Betriebszustand charakterisieren, welcher in Folge der Messung regelbar und/oder steuerbar ist.

Bevorzugt sind die kapillare Struktur und der Flüssigkeitsspeicher mittels einer Verbindungseinrichtung verbunden. Hierdurch kann eine räumliche Trennung der kapillaren Struktur und dem Flüssigkeitsspeicher erreicht werden. Dies kann ein unerwünschtes Erhitzen der Flüssigkeit im Flüssigkeitsspeicher wirksam verhindern.

Vorzugsweise ist die Verbindungseinrichtung dazu eingerichtet, Flüssigkeit mittels Kapillarwirkung zu fördern. Die Förderung der Flüssigkeit in der Verbindungseinrichtung mittels Kapillarwirkung hat die Vorteile, dass die Fördermenge pro Zeit durch die Auslegung der zugrundeliegenden Verbindungseinrichtung eingestellt werden kann, vorzugsweise derart, dass im Betrieb ein genau definierte zu verdampfende Menge an Flüssigkeit gefördert wird, und das eine direkte und kontinuierliche Kopplung zwischen der Verbindungseinrichtung und kapillarer Struktur hergestellt werden kann. Zudem kann auch hier ein separater aktiver Fördermechanismus entfallen.

Vorzugsweise ist die Verbindungseinrichtung ein Vlies. Die das Vlies bildenden Fasern können derart ausgelegt sein, dass die Verbindungseinrichtung eine gewünschte Menge Flüssigkeit aufnehmen und/oder der kapillaren Struktur zuführen kann. Die das Vlies bildenden Fasern können beispielsweise aus Baumwolle, Watte oder Glasfasern sein. Andere Fasern und Kombinationen kommen ebenso in Betracht.

Besonders bevorzugt besteht die Verbindungseinrichtung aus einem elektrisch isolierenden Material. Wenn die kapillare Struktur elektrisch beheizbar ist, wird in dieser Ausführungsform verhindert, dass auch die Verbindungseinrichtung aktiv beheizt wird. Dies wiederum verhindert ein Aufheizen der im Flüssigkeitsspeicher befindlichen Flüssigkeit mit der die Verbindungseinrichtung gekoppelt ist.

Es ist von Vorteil, dass die Verbindungseinrichtung gekapselt ist. Eine Kapselung der Verbindungseinrichtung kann das Verdampfen der Flüssigkeit innerhalb der und/oder auf der Oberfläche der Verbindungseinrichtung verhindern. Die Flüssigkeit wird durch die Kapselung nicht einem Nebenstrom ausgesetzt. Dies trägt so zur zuverlässigen Funktion der kapillaren Struktur bei, in/auf der das Verdampfen stattfinden soll.

Vorzugsweise ist der Flüssigkeitsspeicher ringförmig um den Verdampfer angeordnet. Die ringförmige, vorzugsweise kreisringförmige, Anordnung des Flüssigkeitsspeichers und/oder der Verbindungseinrichtung erlaubt die isotrope Zuführung der Flüssigkeit in die kapillare Struktur und erlaubt daher eine gleichmäßige Versorgung der kapillaren Struktur mit Flüssigkeit aus dem Flüssigkeitsspeicher und damit eine besonders gleichmäßige Verdampfung der Flüssigkeit auf der Oberfläche der kapillaren Struktur.

Des Weiteren kann vorzugsweise stromabwärts von dem Verdampfer ein Filter zum Filtern mindestens der durch den Hauptluftkanal strömenden Luft vorgesehen sein. Der Filter ist vorteilhaft mit einem Abstand hinter dem Verdampferrohr angeordnet.

In einer vorteilhaften Ausführungsform sind das Heizelement und der Verdampfer in einem luftdurchströmten Hauptluftkanal angeordnet, wobei der Hauptluftkanal vorzugsweise durch den Innenraum eines beidseitig offenen Verdampferrohrs gebildet sein kann, vorzugsweise durch ein eintritts- und/oder austrittsseitig frei durchströmbares Verdampferrohr. Erfindungsgemäß weist die Verdampfereinheit einen Nebenluftkanal auf, in dem in die Verdampfereinheit eingesaugte Luft unter Umgehung des Heizelements und des Verdampfers strömt. Vorteilhaft erstreckt sich also der Nebenluftkanal zumindest abschnittsweise parallel zu dem Hauptluftkanal, vorzugsweise konzentrisch. Die Aufteilung des in die Verdampfungseinheit eintretenden Luftstroms in Hauptluftstrom und Nebenluftstrom hat den Vorteil, dass nicht der vollständige eingezogene Luftstrom auf die für die Tröpfchenverdampfung erforderliche Temperatur aufgeheizt werden muss und somit sowohl Heizleistung gespart als auch eine zu hohe Austrittstemperatur vermieden wird. Stromabwärts von dem Verdampfer ist vorzugsweise eine Rückvermischungszone vorgesehen, in der eine erneute Vermischung des erwärmten Hauptluftstroms mit dem Nebenluftstrom erfolgt. Die Verdampfung und die anschließende Rekondensation der Flüssigkeit können durch die Leistung der Luftheizung, die Heizleistung der kapillaren Struktur und die eventuelle Nebenluftführung beeinflusst werden. Dadurch ist eine gezielte Einstellung und Beeinflussung der Tröpfchengröße möglich.

Vorzugsweise ist das Verhältnis des Volumenstroms durch den Hauptluftstrom und den Nebenstrom einstellbar. Die Einstellbarkeit der Volumenströme durch den Hauptluftkanal und den Nebenluftkanal kann die Funktion der Verdampfereinheit positiv beeinflussen, da Strömungsgeschwindigkeiten und Wärmetransport von den Volumenströmen abhängen. Die Einstellbarkeit ist vorzugsweise aktiv und/oder passiv vornehmbar. Aktive Einstellbarkeit bedeutet Einstellbarkeit, welche durch eine Steuerung und/oder Regelung die durch eine elektronische/elektrische Einheit realisierbar ist. Passive Einstellbarkeit bedeutet Einstellbarkeit, welche durch ein einmaliges Einstellen, beispielsweise durch Verstellmechanismen oder Wechselbauteile, realisierbar ist.

Es sind Ausführungsformen ohne Nebenluftkanal denkbar, wobei dann die gesamte durch die Verdampfereinheit strömende Luft durch den Hauptluftkanal strömt.

Besonders vorteilhaft ist der Hauptluftkanal innerhalb des Nebenluftkanals angeordnet. Dies ist energetisch günstig, weil der Nebenluftkanal den Hauptkanal nach außen thermisch isoliert. Insbesondere können der Hauptluftkanal und der Nebenluftkanal konzentrisch zueinander angeordnet sein.

Vorteilhaft ist der Nebenluftkanal benachbart, vorzugsweise beabstandet zu dem Hauptluftkanal angeordnet. Diese Ausführungsform erlaubt ein breites Spektrum an geometrischen Anordnungsformen der Erfindung.

Vorzugsweise ist der Strömungsquerschnitt des Nebenluftkanals größer oder kleiner als der Strömungsquerschnitt des Hauptluftkanals, insbesondere am Eintritt und/oder Austritt des Nebenluftkanals. Der mit verdampfter Flüssigkeit nach dem Verdampfer angereicherte Luftstrom ist vorteilhaft mit gleichbleibender oder abnehmender Temperatur in der Verdampfereinheit geführt. Vorzugsweise weist der Nebenluftkanal einen Eintritt und einen Austritt auf, wobei bevorzugt der Eintritt vor dem Heizelement und/oder vor dem Verdampfer und/oder der Austritt nach dem Heizelement und/oder nach dem Verdampfer angeordnet sind.

Ein Vorsehen des Nebenluftkanals ermöglicht es zudem, die Außentemperatur der Verdampfungseinheit und eines damit ausgestatteten Inhalators niedrig zu halten und die "heiße Verdampfungszone" innen zu konzentrieren, wodurch evtl. Verbrennungsgefahr beim Halten des Inhalators minimiert wird. Der Nebenluftkanal ermöglicht auf optimale Art und Weise, ein gewünschtes Tropfenspektrum einzustellen, Abwärme aus dem Hauptluftstrom zu nutzen und eine effiziente Mischzone mit optimalen Mischungsvoraussetzungen zu realisieren.

Nach einem weiteren Aspekt der Erfindung wird ein Verfahren zum Steuern einer zuvor beschriebenen Verdampfereinheit bereitgestellt.

Vorteilhaft wird die kapillare Struktur und/oder das Heizelement nicht-kontinuierlich, insbesondere zyklisch und/oder gepulst, beheizt. Dies kann den Energieeintrag in das Heizelement und ggf. die kapillare Struktur optimieren und erlaubt das bedarfsgerechte Heizen, wenn eine Verdampfung erzielt werden soll sowie eine Regelung und Steuerung der Heizleistung, und kann der Nachförderung der Flüssigkeit dienlich sein. Vorteilhaft ist, dass der zeitliche Heizspannungsverlauf Uhk(t) oder die zeitlichen Heizspannungsverläufe Uh(t) für das Heizelement und Uhk(t) für die kapillare Struktur so vorgegeben ist/sind, dass ein Nachströmen von Flüssigkeit in Abhängigkeit von der Temperatur gewährleistet ist. Abhängig von der Förderleistung und/oder des Förderbedarfs kann so die Heizleistung eingestellt werden. Die Menge des nachgeförderten Liquids entspricht vorzugsweise der verdampften Menge des Liquids. Es strömt also nur das nach, was verdampft wurde. Ein Einstellten der Temperatur ist in jedem Falle denkbar. Weiter bevorzugt wird die Heizleistung des Heizelements und/oder der kapillaren Struktur an einer

Temperaturobergrenze abgeregelt. Dies erlaubt ein zuverlässiges Verdampfen unter der Maßgabe, dass keine Überhitzung und/oder schädliche thermische Zersetzung der zu verdampfenden Flüssigkeit und/oder des Dampfes auftritt. Besonders bevorzugt ist eine geführte Verdampfung nach Temperaturvorgaben gewährleistet. Mit einem der genannten Verfahren lässt sich ein optimales Verdampfen der Flüssigkeit, angepasst an deren Komponenten gewährleisten und ein Entstehen unerwünschter Zersetzungsprodukte zuverlässig vermeiden.

Die Erfindung wird im Folgenden anhand bevorzugter Ausführungsformen unter Bezugnahme auf die beigefügten Figuren erläutert. Dabei zeigt
- Fig. 1: eine schematische Querschnittsansicht eines elektronischen Zigarettenprodukts in einer Ausführungsform der Erfindung;
- Fig. 2: eine schematische Darstellung einer erfindungsgemäßen Verdampfereinheit mit einer ersten elektrischen/elektronischen Einheit;
- Fig. 3: eine schematische Darstellung einer erfindungsgemäßen Verdampfereinheit mit einer ersten und einer zweiten elektrischen/elektronischen Einheit;
- Fig. 4: einen schematischen Schnitt durch eine Verdampfereinheit mit einem um diese konzentrisch angeordneten Flüssigkeitsspeicher;
- Fig. 5: eine schematische Querschnittsansicht einer Mikrofibrille für eine bionische Struktur; und
- Fig. 6: eine aus Mikrofibrillen gemäß Figur 5 aufgebaute bionische Heizstruktur.

Das elektronische Zigarettenprodukt 10 umfasst ein im Wesentlichen stabförmiges oder zylindrisches Gehäuse 11. In dem Gehäuse 11 ist ein Luftkanal 30 zwischen mindestens einer Lufteinlassöffnung 31 und dem Mundende 32 des Zigarettenprodukts 10 vorgesehen. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und eine Luftströmung 34 in dem Luftkanal 30 erzeugt. Mindestens eine Lufteinlassöffnung 31 kann an der Mantelseite des Gehäuses 11 angeordnet sein. Zusätzlich oder alternativ kann mindestens eine Lufteinlassöffnung 31A am entfernten Ende 33 des Zigarettenprodukts 10 angeordnet sein. Das entfernte Ende 33 bezeichnet das dem Mundende 32 entgegengesetzte Ende des Zigarettenprodukts 10.

Nach einer oder beiden Lufteinlässen 31, 31A ist im Strömungsweg der Luftströmung 34 eine Lufterwärmungseinrichtung 37 zum Erwärmen bzw. Vorwärmen der eintretenden Luft angeordnet. Hierdurch kann die Aerosolbildung optimiert werden. Die Lufterwärmungseinrichtung 37 kann beispielsweise benachbart zu einer Energieversorgungseinheit 14 angeordnet sein, sich in Umfangsrichtung um die Mantelinnenseite des Gehäuses 11 erstrecken, einen Heiter umfassen und/oder ist vorteilhafterweise konzentrisch angeordnet.

Die durch die Einlassöffnung 31 angesaugte Luft wird in dem Luftkanal 30, ggf. über die Schnittstelle bzw. Trennfläche 57 zu einer Verdampfereinheit 20 geleitet. Die Verdampfereinheit 20 gibt Flüssigkeit 50 aus dem Flüssigkeitsspeicher 18 als Zugabe in Form kleiner Flüssigkeitstropfen als Nebel/Aerosol und/ oder gasförmig als Dampf in den Luftstrom 34 zu. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml. Der Flüssigkeitsspeicher 18 weist vorzugsweise eine geschlossene Oberfläche auf und ist vorzugsweise ein flexibler Beutel, um Flüssigkeitsleckage zu vermeiden, und umfasst somit einen Liquidtank. Die Liquidzufuhr ergibt sich vorteilhaft durch die verdampfe Flüssigkeitsmenge, d.h. ist ein Auslegungsparameter der kapillaren Struktur 40.

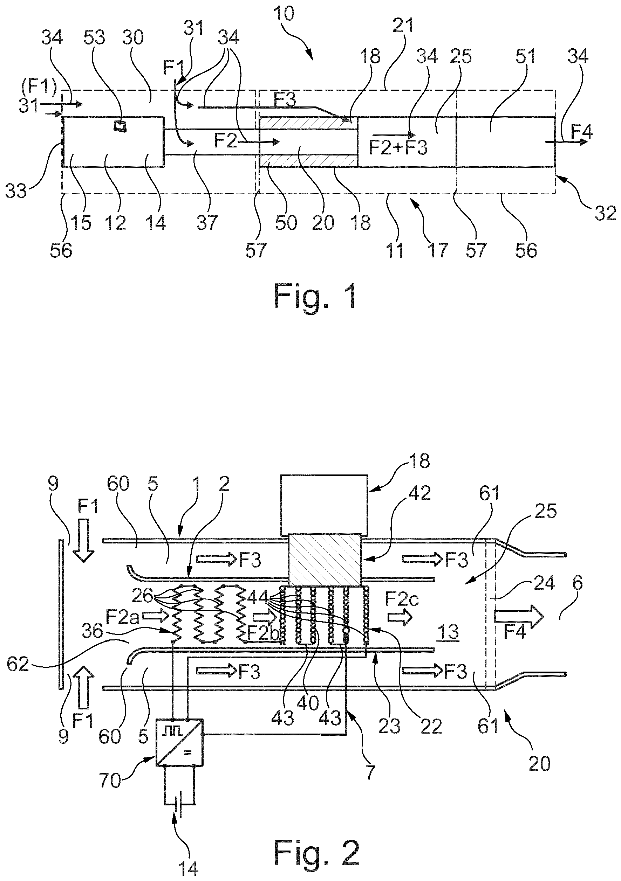

Die geometrischen Abmessungen und die Anordnung der Verdampfereinheit 20 in dem Inhalator 10 bzw. der Verbrauchseinheit 17 gemäß Figur 1 ist schematisch und nicht maßstabsgetreu. Insbesondere kann die Verdampfereinheit 20 wesentlich länger sein als in Figur 1 gezeigt und sich beispielsweise über mindestens die halbe Länge des Inhalators 10 erstrecken.

Das Zigarettenprodukt 10 umfasst, vorteilhaft am entfernten Ende 33 des Zigarettenprodukts 10, die elektronische Energieversorgungseinheit 12 mit einem elektrischen Energiespeicher 14 und einer elektrischen/elektronischen Einheit 15, das heißt beispielsweise einem Akku und einer Steuerung. Der Energiespeicher 14 kann insbesondere eine elektrochemische Einweg-Batterie oder ein wiederaufladbarer elektrochemischer Akku, z.B. ein Li-Ionen-Akku, sein. Das Zigarettenprodukt 10 umfasst des Weiteren eine Verbrauchseinheit 17 mit einem Flüssigkeitsspeicher 18und der Verdampfereinheit 20. Eine elektrischen/elektronischen Einheit 19 (nicht gezeigt) kann ebenso in der Verbrauchseinheit 17 angeordnet sein.

Anstelle der getrennten elektrischen/elektronischen Einheiten 15, 19 kann auch eine einheitliche elektrische/elektronische Einheit vorgesehen sein, die entweder in der Energieversorgungseinheit 12 oder in der Verbrauchseinheit 17 angeordnet sein kann. Die Gesamtheit der elektrischen/elektronischen Einheit(en) des Zigarettenprodukts 10 wird im Folgenden als Steueranordnung 29 bezeichnet.

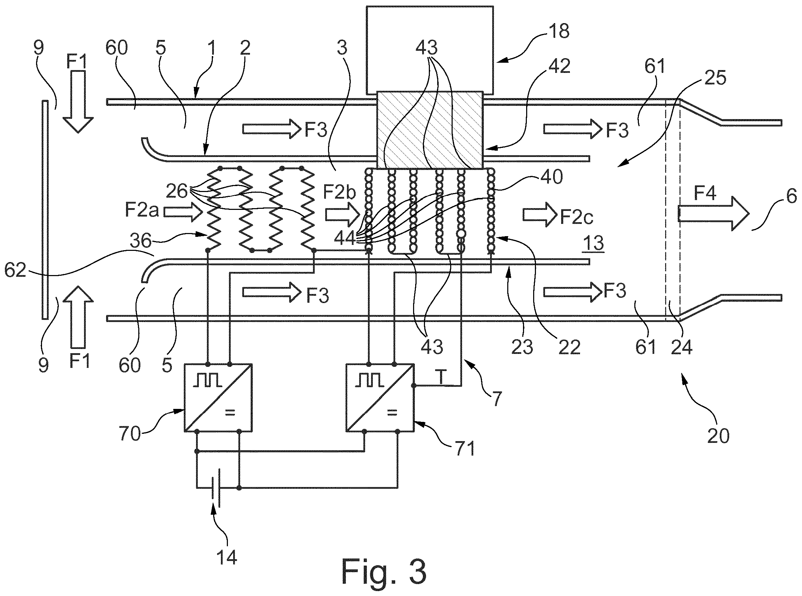

In dem Gehäuse 11 ist vorteilhaft mindestens ein Sensor 7, beispielsweise ein Temperatursensor, angeordnet, dessen Funktion später erläutert wird, siehe Figuren 2 und 3. Zusätzlich oder alternativ kann ein Drucksensor oder ein Druck- oder Strömungsschalter vorgesehen sein, wobei die Steueranordnung 29 auf der Grundlage eines von dem Sensor ausgegebenen Sensorsignals einen Betriebszustand des Zigarettenprodukts 10, in dem ein Konsument am Mundende 32 des Zigarettenprodukts 10 zieht, um zu inhalieren, feststellen kann. In diesem Betriebszustand steuert die Steueranordnung 29 die Verdampfereinheit 20 an, um Flüssigkeit 50 aus dem Flüssigkeitsspeicher 18 als Zugabe in Form kleiner Flüssigkeitstropfen als Nebel/Aerosol und/oder gasförmig als Dampf in den Luftstrom 34 zuzugeben. Die Flüssigkeitszugabe geschieht vorzugsweise nicht aktiv.

Die in dem Flüssigkeitsspeicher 18 gespeicherte, zu dosierende Flüssigkeit (d.h. das flüssige Komponentengemisch) ist beispielsweise eine Mischung aus 1,2-Propylenglykol, Glycerin und/oder Wasser, der ein oder mehrere Aromen (Flavour) und/oder Wirkstoffe, wie beispielsweise Nikotin, zugemischt sein können.

Die Verbrauchseinheit 17 ist vorteilhaft als vom Konsumenten auswechselbare Kartusche 21, d.h. als Einwegteil ausgeführt. Der insbesondere den Energiespeicher 14 enthaltende Rest des Zigarettenprodukts 10 ist vorteilhaft als vom Konsumenten wiederverwendbares Grundteil 56, d.h. als Mehrwegteil ausgeführt. Die Kartusche 21 ist vom Konsumenten mit dem Grundteil 56 verbindbar und vom Grundteil 56 lösbar ausgebildet. Zwischen der Kartusche 21 und dem wiederverwendbaren Grundteil 56 ist somit eine Trennfläche bzw. Schnittstelle 57 gebildet. Das Kartuschengehäuse 58 kann einen Teil des Gehäuses 11 des Zigarettenprodukts 10 bilden.

In anderen nicht gezeigten Ausführungsformen ist die Verbrauchseinheit 17 als Kartusche 21 ausgeführt, die in den wiederverwendbaren Grundteil 56 des Zigarettenprodukts 10 durch den Konsumenten einsetzbar und aus diesem entnehmbar ist. Das Kartuschengehäuse ist in diesem Fall ein von dem Gehäuse 11 des Zigarettenprodukts 10 separates Gehäuse.

Die Kartusche 21 umfasst mindestens den Flüssigkeitsspeicher 18. Die Kartusche 21 kann die elektrische/elektronische Einheit 19 umfassen. In anderen Ausführungsformen ist die elektrische/elektronische Einheit 19 ganz oder teilweise fester Bestandteil des Grundteils 56. Ebenso kann die Verdampfereinheit 20 Teil der Kartusche 21 oder in dem Grundteil 56 angeordnet sein. Die Kartusche 21 kann daher in manchen Ausführungsformen im Wesentlichen nur aus dem Flüssigkeitsspeicher 18 und ggf. dem Kartuschengehäuse bestehen. Das Kartuschengehäuse kann alternativ von dem Gehäuse des Flüssigkeitsspeichers 18 gebildet sein, so dass ein separates Kartuschengehäuse entbehrlich sein kann.

Die Kartusche 21 kann neben der Verwendung in stabförmigen Zigarettenprodukten 10 auch in anderen Inhalatoren eingesetzt werden, beispielsweise in elektronischen Pfeifen, Shishas, anderen Heatnot-burn-Produkten, oder einem medizinischen Inhalator. Der Energiespeicher 14 ist in der Regel nicht Teil der Kartusche 21, sondern Teil des wiederverwendbaren Grundteils 56.

Die Verbrauchseinheit 17 bzw. die Kartusche 21 umfasst vorteilhaft einen nichtflüchtigen Informationsspeicher 53 (siehe Fig. 1) zum Speichern von die Verbrauchseinheit 17 (einen ID-Chip) bzw. die Kartusche 21 betreffender Information bzw. Parametern, beispielsweise in Ausführung als EEPROM, RFID oder anderer geeigneter Form. Der Informationsspeicher 53 kann Teil der elektrischen/elektronischen Einheit 19 oder separat davon ausgebildet sein. In dem Informationsspeicher 53 gespeichert ist vorteilhaft Information zum Inhaltsstoff, d.h. zur Zusammensetzung der in dem Flüssigkeitsspeicher 18 gespeicherten Flüssigkeit; Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, insbesondere umfassend eine ID zur eindeutigen Kennzeichnung der Verbrauchseinheit 17 bzw. Kartusche 21; Seriennummer, Herstelldatum und/oder Ablaufdatum; und/oder Zugzahl (Anzahl der Inhalationszüge durch den Konsumenten) bzw. der Nutzungszeit. Der Datenspeicher 53 ist vorteilhaft über Kontakte und/oder Leitungen mit der Steuereinrichtung 15 des Grundteils 56 verbunden oder verbindbar.

Eine vorteilhafte Ausführungsform einer erfindungsgemäßen Verdampfereinheit 20 ist in Fig. 2 gezeigt.

Die Verdampfereinheit 20 kann ein eigenes Gehäuse 1 aufweisen, das Gehäuse 1 kann jedoch alternativ ganz oder teilweise von dem Gehäuse 11 des Zigarettenprodukts gebildet werden. Die Verdampfereinheit weist mindestens eine Lufteingangsöffnung 9 für den Zustrom F1 der durch die Verdampfereinheit 20 durchzusetzenden Luft und mindestens eine Luftausgangsöffnung 6 auf, wobei die Luftströmung 34, die in Figur 2 mit F1 bis F4 bezeichnet wird, von der Lufteingangsöffnung 9 zu der Luftausgangsöffnung 6 durch die Verdampfereinheit 20 strömt.

Innerhalb der Verdampfereinheit 20 ist der Luftkanal 30 in zwei parallel angeordnete Kanäle aufgeteilt, und zwar in einen Hauptluft- oder Heizkanal 3, in dem ein Heizelement 36 angeordnet ist, und einen Nebenluft- oder Bypasskanal 5, in dem ein Nebenluftstrom F3 unter Umgehung der Heizelements 36 strömen kann. Demnach wird die in die Verdampfereinheit eintretende, ggf. vorgewärmte Luftströmung F1 in einen Hauptluftstrom F2a und einen Nebenluftstrom F3 aufgeteilt. Anders gesagt wird ein Teil des Luftstroms F1 als Nebenstrom F3 um den Verdampfer 22 herumgeführt, ein anderer Teil des Luftstroms F1 wird als Hauptluftstrom F2 durch den Verdampfer 22 hindurchgeführt und vom Nebenstrom durch das Verdampferrohr 2 getrennt.

Der Nebenluftkanal 5 weist einen Eintritt 60 und einen Austritt 61 auf. Der Eintritt 60 ist vor dem Heizelement 36 und/oder vor dem Verdampfer 22 angeordnet. Der Austritt 61 ist nach dem Heizelement 36 und/oder nach dem Verdampfer 22 angeordnet. Vorzugsweise steht der Hauptluftkanal 3 mit einer Hauptluftzutrittsöffnung 62 in Verbindung, welche dem Heizelement 36 vorgeordnet ist. Der Nebenluftkanal 5 steht vorteilhaft mit der Nebenluftzutrittsöffnung 60 in Verbindung, welche der Hauptluftzutrittsöffnung 62 vorgeordnet oder nachgeordnet ist. Die Nebenluftzutrittsöffnung 62 ist dem Verdampfer 22 vorgeordnet oder nachgeordnet.

Die Aufteilung des in die Verdampfungseinheit 20 eintretenden Luftstroms F1 in Hauptluftstrom F2 und Nebenluftstrom F3 hat den Vorteil, dass nicht der vollständige eingezogene Luftstrom F1 auf die für die Tröpfchenverdampfung erforderliche Temperatur aufgeheizt werden muss und somit sowohl Heizleistung gespart als auch eine zu hohe Austrittstemperatur vermieden wird.

Der Hauptkanal 3 ist vorteilhaft radial innen und der der Nebenluftkanal 5 radial außen in der Verdampfereinheit 20 angeordnet, d.h. konzentrisch, so dass der Nebenluftkanal 5 den Hauptkanal 3 vorteilhaft umfangsseitig umschließt. Dies ist energetisch günstig, weil der Nebenluftkanal 5 den Hauptkanal 3 nach außen thermisch isoliert. Der Hauptkanal 5 wird vorteilhaft durch den Innenraum eines Verdampferrohrs 2 gebildet. Der Nebenluftkanal 5 ist vorteilhaft ein Ringkanal, der insbesondere zwischen dem Strömungsrohr 2 und dem Gehäuse 1 gebildet ist. Vorzugsweise sind Nebenluftkanal 5 und der Hauptkanal 3 konzentrisch zueinander angeordnet. Das Gehäuse 1 kann die äußere Strömungsführung für den Nebenluftkanal 5 übernehmen.

In dem Hauptkanal 3 ist stromabwärts von dem Heizelement 36 ein Verdampfer 22 zur Zugabe von aus dem Flüssigkeitsspeicher 18 stammender Flüssigkeit und Beimischung zu dem durch das Heizelement 36 erhitzten Hauptluftstrom F2b vorgesehen. Der oben beschriebene Verdampfer 22 weist zu diesem Zweck eine kapillare Struktur 40 auf. Die kapillare Struktur 40 umfasst vorzugsweise mindestens ein Strukturelement 44, vorzugsweise eine Mehrzahl von Strukturelementen 44, welche vorteilhaft durch Leitungselemente 43 miteinander elektrisch verbunden sind. Eine Mehrzahl von Strukturelementen 44 ist vorzugsweise in Strömungsrichtung des Luftstroms F2b hintereinander angeordnet. In den Ausführungsbeispielen der Figuren 2 und 3 ist eine Mehrzahl von beispielsweise sechs angeordneten Strukturelementen 44 vorgesehen. Die Strukturelementen 44 sind vorteilhaft durch Leitungselemente 43 in Serie miteinander verschalteten. Die Strukturelemente 44 können gitterförmig, mäanderförmig, wendelförmig, spiralförmig, mesh-förmig, vliesartig oder gewebeartig sein, insbesondere in Form eines gefalteten oder gerollten Metallgewebes, eine mikromechanisch hergestellte Struktur aus Kanälen und Rippen und/oder ihrer Form und Struktur einer bionischen Struktur, wie beispielsweise Mikrofibrillen 85, nachempfunden sein. Um im Bereich der kapillaren Struktur 40 Schadstoffentstehung durch katalytische Effekte und/oder Metallfreisetzung in den Dampf/das Aerosol hinein zu vermeiden, kann die kapillare Struktur 40 auch metallfrei, ggf. als Silizium, als polymeres Silizium, keramisch, metallisch, halbmetallisch oder als aus Graphit bestehend ausgeführt sein.

Die kapillare Struktur 40 erzeugt eine Kapillarwirkung, d.h. verdampfte Flüssigkeit wird selbsttätig in der kapillaren Struktur 40 nachgefördert. Die kapillare Struktur 40 ist dabei nicht auf längliche Kapillaren beschränkt, sondern kann Poren oder sonstige Hohlräume aufweisen, die einen Kapillareffekt bereitstellen. Im Besonderen können vorteilhaft auch bionische Strukturen, wie beispielsweise Mikrofibrillen 85, eingesetzt werden. Vorteilhaft weisen das mindestens eine Strukturelement 44 und somit die kapillare Struktur 40 des Verdampfers 22 eine Porosität auf, vorzugsweise eine hohe Porosität, welche durch die Maschenweite und/oder die Größe der Poren, Kanäle oder sonstiger Hohlräume der kapillaren Struktur bestimmt ist. Eine hohe Porosität bedingt eine große Oberfläche 41, welche mit Flüssigkeit benetzbar ist und welche dann verdampf werden kann. Die Porosität gewährleistet des Weiteren die erforderliche Luftdurchlässigkeit der Strukturelemente 44 und somit die der kapillaren Struktur 40, sowie die vorgesehene Kapillarwirkung zur Förderung der Flüssigkeit. Eine Mehrzahl von Strukturelementen 44 kann parallel und/oder winkelig zueinander angeordnet sein. Das mindestens eine Strukturelement 44 der kapillaren Struktur 40 kann eine räumlich periodische, aperiodische, isotrope und/oder anisotrope Struktur aufweisen.

Bei Feststellung eines durch Ziehen des Konsumenten verursachten Luftstroms 34 durch den Luftkanal 30 steuert die Steuereinrichtung 29 das Heizelement 36 und die beheizbare kapillare Struktur 40 an, um die Verdampfungseinheit in Betrieb zu setzen. Dies wird im Folgenden genauer beschrieben.

Der Hauptluftstrom F2a durchströmt zuerst das elektrische Heizelement 36, das die Luft auf eine geeignete Temperatur erwärmt.

Das Heizelement 36 wird durch die erste elektrische/elektronische Einheit 70, die vorzugsweise eine Leistungselektronik umfasst, gesteuert oder geregelt, beispielsweise durch Pulsweitenmodulierung. Eine von der Heizspannungsquelle 14 erzeugte elektrische Spannung Uh wird an das Heizelement 36 angelegt und führt zu einem Stromfluss durch das Heizelement 36. Dieser führt aufgrund des ohmschen Widerstands des Heizelements 36 zu einer Erhitzung des Heizelements und daher zu einer Erwärmung der an dem Heizelement 36 vorbeiströmenden Luft F2a. Das Heizelement 36 umfasst vorzugsweise mindestens eine Heizstruktur 26, hier eine Mehrzahl von beispielsweise vier in Serie miteinander verschalteten Heizstrukturen 26. Auch eine Gitterstruktur ist denkbar.

In einer bevorzugten Ausführungsform ist auch die kapillare Struktur 40 beheizbar. Die kapillare Struktur 40 wird in der in Figur 2 gezeigten Ausführungsform ebenfalls durch die erste elektrische/elektronische Einheit 70, die vorzugsweise eine Leistungselektronik umfasst, gesteuert oder geregelt, beispielsweise durch Pulsweitenmodulierung. Eine von der Heizspannungsquelle 14 erzeugte elektrische Spannung Uhk wird angelegt und führt zu einem Stromfluss durch das Heizelement 36 und die kapillare Struktur 40. Dieser führt aufgrund des ohmschen Widerstands des Heizelements 36 und dem der kapillaren Struktur 40 zu einer Erhitzung des Heizelements 36 und der kapillaren Struktur 40 und daher zu einer Erwärmung der an dem Heizelement 36 vorbeiströmenden Luft F2a, welche auf die erwärmte kapillare Struktur 40 trifft, um diese zu umströmen und/oder zu durchströmen und zur Verdampfung beizutragen.

Die geeignete Einstellung der ohmschen Widerstände der Strukturelemente 44 und der Leitungselemente 43 kann dazu dienen, verschiedene Strukturelemente 44 auf gleiche oder verschiedene Temperaturen zu erhitzen. Beispielsweise können die Strukturelemente 44 stromabwärts stärker beheizt werden um einer durch Verdampfungswärme und Diffusion bedingten Abkühlung innerhalb des Verdampfers entgegenzuwirken. Dadurch wird eine besonders gleichmäßige Verdampfung erzielt und eine unerwünschte Kondensation bereits verdampfter Flüssigkeit kann verhindert werden. Vorzugsweise steht der in oder an der kapillaren Struktur 40 befindliche Sensor 7 zur Messung von Betriebsbedingungen, wie beispielsweise der Temperatur, der kapillaren Struktur 40 im Kontakt zu der ersten elektrischen/elektronischen Steuereinheit 70 und/oder zu anderen Steuerelementen, welche die Beheizung der kapillaren Struktur 40 regeln und/oder steuern. Beispielsweise kann so eine Ein- oder Abschaltung der Heizleistung der kapillaren Struktur 40 und/oder des Heizelements 36 bei einer gegebenen Temperatur erreicht werden. Eine andere Steuerung/Regelung aufgrund vom Sensor 7 erfasster Daten ist ebenfalls möglich, im Besonderen die Temperaturregelung. Auch kann eine Mehrzahl von Sensoren 7 in den jeweiligen Strukturelementen 44 oder an anderen Orten des Verdampfers 22 vorgesehen sein, um beispielsweise Temperatur-, Druck,- oder Konzentrationsgradienten zu bestimmen und entsprechende Steuerungs- und/oder Regelungsmaßnahmen vorzunehmen. Auch können Sensoren in/an dem Heizelement 36 vorgesehen sein oder von diesem gebildet werden.

Der zeitliche Heizspannungsverlauf Uhk(t) für die kapillare Struktur kann vorteilhaft so vorgegeben werden, dass ein Nachströmen von Flüssigkeit gewährleistet ist.

Die Temperatur der erwärmten Luftströmung F2b kann beispielsweise geringfügig, um bis zu 40 °C, oberhalb oder unterhalb der Siedetemperatur des Liquids liegen, das diese nach der Verdampfung einer vorgegebenen Teilmenge aufweist. Damit ist sichergestellt, dass eine zügige Verdampfung der Tröpfchen bis zu diesem Zustand erfolgen kann und die Verdampfung nicht zu langsam erfolgt. Vorteilhaft soll die Luft F2b nicht stärker erwärmt werden, als die Vermeidung thermischer Zersetzung der Komponenten zulässt.

Der erwärmte Luftstrom kann optional durch ein hier nicht dargestelltes Strömungsvergleichmäßigungselement, beispielsweise in Form eines Gitters oder von Drallelementen, bezüglich seines Strömungs- und Temperaturprofils homogenisiert werden. Das Strömungsvergleichmäßigungselement ist vorzugsweise in dem Hauptluftkanal 3 und weiter vorzugsweise zwischen dem Heizelement 36 und dem Verdampfer 22 angeordnet.

Der erwärmte Luftstrom F2b trifft auf das in der kapillaren Struktur 40 vorgewärmte Liquid und verdampft eine bestimme Menge des Liquids, welche durch Lufttemperatur, Volumenstrom sowie die (Förder-)Eigenschaften der kapillaren Struktur 40 einstellbar ist. Das verdampfte Liquid strömt weiter als Dampf oder Aerosol längs des Luftstroms F2c. Die Anordnung und Ausrichtung der kapillaren Struktur 40 kann längs mit dem Luftstrom F2b oder quer dazu erfolgen, sofern ein gezieltes Verdampfen vor dem oder im Luftstrom F2c zu gewährleistet ist.

Nach der Zugabe aus dem Verdampfer 22 wird die auf der Oberfläche 41 der kapillaren Struktur 40 befindliche, vorteilhaft vorgewärmte Flüssigkeit, d.h. der Flüssigkeitsfilm, durch den Kontakt mit dem heißen Luftstrom F2b im Wesentlichen instantan verdampft. Auf diese Weise ist in der Verdampfereinheit 20 bzw. in dem Verdampferrohr 2 oder Hauptkanal 3 ein Verdampfer 22 gebildet. In der nach dem Verdampfer 22 liegenden Zone 13 findet weiter die Bildung des Luft/Aerosolstroms F2c statt.

Die Vermischung des dampfhaltigen Hauptluftstroms F2c mit dem Nebenluftstrom F3 erfolgt in der Rückvermischungszone bzw. Mischstrecke 25. Vorteilhaft wird dabei der angereicherte Kernstrom von den kühlen Wänden im Austritt ferngehalten und Wandkondensation vermieden. Die vereinte dampfhaltige Luftströmung F4 verlässt die Verdampfereinheit 20 vorzugweise schließlich durch eine Kühlstrecke 51 und die Ausgangsöffnung 6.

Die Verbindungseinrichtung 42, welche den Verdampfer 22 mit dem Flüssigkeitsspeicher 18 verbindet, kann durch ein elektrisch nichtleitendes Vlies gebildet sein. In dieser Ausführungsform kann das Vlies mit der kapillaren Struktur 40 mechanisch bzw. flüssigkeitsleitend gekoppelt sein und so einen durch Kapillarwirkung bedingten Transport der Flüssigkeit aus dem Flüssigkeitsspeicher 18 über die Verbindungseinrichtung 42 in die kapillare Struktur 40 ermöglichen. Im Besonderen können dem Vlies die gleichen Materialeigenschaften und Herstellungsschritte zugrunde liegen wie den Strukturelementen 44 der kapillaren Struktur 40. Andere Verbindungseinrichtungen 42 sind jedoch auch denkbar und können durch eine gesonderte Durchflusssteuereinrichtung, z.B. eine Mikropumpe oder ein Ventil, mit dem erforderlichen Liquidvolumenstrom aus dem Flüssigkeitsspeicher 18 beaufschlagt werden. Bei Einsatz einer Ventilstruktur mit einem oder mehreren Ventilen kann der Flüssigkeitsspeicher 18 vorteilhaft druckbeaufschlagt sein.

Der Flüssigkeitsspeicher 18, die Verbindungseinrichtung 42, die erste elektrische/elektronische Einheit 70 sowie die ggf. vorhandene zweite elektrische/elektronische Einheit 70 und die Stromversorgung 14 können in unterschiedlichen räumlichen Konfigurationen realisiert werden. Des Weiteren ist eine zusätzliche geregelte elektrische Vorwärmung des zu verdampfenden Liquids realisierbar, wodurch der überwiegende Teil der zur Verdampfung erforderlichen Wärme eingebracht werden kann und/oder die Liquideigenschaften (Oberflächenspannung / Viskosität) beeinflusst werden können, was für den Tröpfchenbildungsprozess sowie den Förderprozess relevant ist. Diese Vorwärmung kann in dem Verdampfer 22 oder in der Verbindungseinrichtung 42 integriert sein.

Die Regelung der Temperatur des Hauptluftstroms F2b kann über die Messgröße des elektrischen Widerstands des Heizelementes erfolgen, sofern dieser temperaturabhängig ist, oder direkt mit Hilfe eines optionalen Sensors 7 im homogenisierten Hauptluftstrom F2b.

Vorzugsweise ist in dem Datenspeicher 53 der Kartusche 21 oder in einem Datenspeicher 59 des Grundteils 56 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass die Heiztemperatur des Heizelements 36 gemäß der exakt bekannten Verdampfungskinetik des jeweiligen Liquids zeitlich über den Verdampfungsvorgang steuerbar ist, wodurch optimale Verdampfungsergebnisse erzielbar sind. Die Verdampfungstemperatur liegt vorzugsweise im Bereich zwischen 100 °C und 400 °C, weiter bevorzugt zwischen 150 °C und 350 °C, noch weiter bevorzugt zwischen 190 °C und 240 °C.

Die Verdampfungseinheit 20 ist so eingestellt, dass eine vorteilhafte Flüssigkeitsmenge im Bereich zwischen 1 µl und 20µl, weiter vorzugsweise zwischen 2µl und 10µl, noch weiter vorzugsweise zwischen 3µl und 5µl, typischerweise 4µl pro Zug des Konsumenten, zudosiert wird. Vorzugsweise kann die Verdampfungseinheit 20 hinsichtlich der Flüssigkeitsmenge pro Zug einstellbar sein.

Der Verdampfer 22 kann vorteilhaft so eingestellt sein, dass im Aerosol überwiegend Flüssigkeitstropfen mit einem Durchmesser im Bereich zwischen 0,05 µm und 5 µm, bevorzugt zwischen 0,1 µm und 3 µm entstehen. Tröpfchengrößen im Bereich zwischen 0,05 und 5 MMAD (mass median aerodynamic diameter, massenmedianer aerodynamischer Durchmesser), vorzugsweise zwischen 0,1 und 3 MMAD, weiter vorzugsweise zwischen 0,3 und 2 MMAD, noch weiter vorzugsweise zwischen 0,4 und 1,5 MMAD, beispielsweise um ca. 0.5 MMAD oder 1 MMAD können optimal sein. MMAD entspricht einer EU-Norm und wird in µm spezifiziert.

Das Heizelement 36 und/oder der Verdampfer 22 werden vorteilhaft mit einer geeigneten Ansteuerfrequenz typischerweise im Hz- oder kHz-Bereich und beispielsweise zwischen 1 Hz und 50 kHz, bevorzugt zwischen 30 Hz und 30 kHz, besonders bevorzugt zwischen 100 Hz und 25 kHz elektrisch angesteuert. In einer alternativen Ausgestaltung kann die Ansteuerfrequenz im Bereich zwischen 5 Hz und 50 Hz, bevorzugt zwischen 10 Hz und 40 Hz liegen.

In einer weiteren Ausführungsform können Heizelement 36 und/oder Verdampfer 22 kontinuierlich angesteuert und/oder geregelt werden, insbesondere über Teilabschnitte eines Zugs, Puffs und/oder eine Serie aufeinanderfolgender Züge bzw. Puffs.

Eine alternative Ausführungsform der Erfindung ist in Figur 3 zu sehen. Diese Ausführungsform hat die Eigenschaften der in Figur 2 gezeigten Ausführungsform, unterscheidet sich jedoch in der Anwesenheit von einer separaten zweiten elektrischen/elektronischen Einheit 71 zur Steuerung der Heizung der kapillaren Struktur 40. Diese Ausführungsform erlaubt eine gezieltere Steuerung und/oder Regelung des Heizelements 36 und der kapillaren Struktur 40. Analog zur ersten elektrischen/elektronischen Einheit 70 ist diese vorteilhaft zeitveränderlich gesteuert oder geregelt, beispielsweise durch Pulsweitenmodulierung. Eine von der Heizspannungsquelle 14 erzeugte elektrische Spannung Uhk wird angelegt und führt zu einem Stromfluss durch die kapillare Struktur 40. Dieser führt aufgrund des ohmschen Widerstands der kapillaren Struktur 40 zu einer Erhitzung der kapillaren Struktur. Eine zweite Energieversorgungseinheit ist denkbar. Weitere Vorgänge und Bestandteile sind analog zur in Figur 2 gezeigten Ausführungsform.

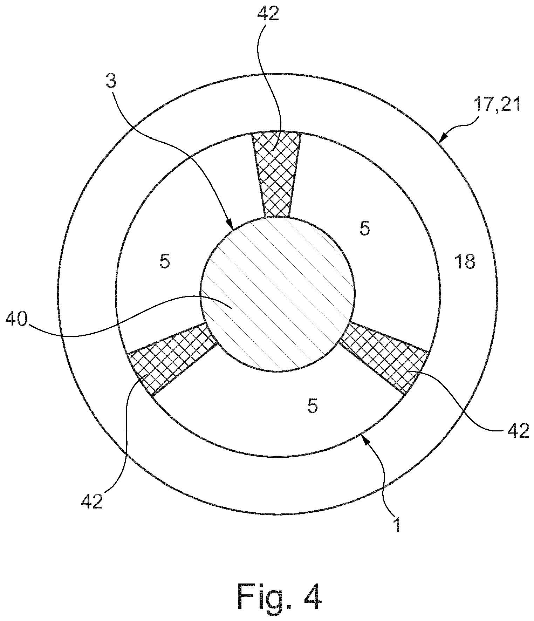

Ein Schnitt einer bevorzugten Ausführungsform der Erfindung ist in Figur 4 zu sehen. Die Verbrauchseinheit 17 umfasst einen Flüssigkeitsspeicher 18. Der Flüssigkeitsspeicher 18 ist vorteilhaft ringförmig um das Gehäuse 1 angeordnet, in welchem sich vorzugsweise der Nebenluftkanal 5 befindet. Der Nebenluftkanal 5 umschließt vorzugsweise ringförmig den Hauptluftkanal 3, in welchem sich die kapillare Struktur 40 befindet. Die Verbindungseinrichtung 42 umfasst vorteilhaft eine Mehrzahl von Elementen, in Figur 4 drei Elemente, welche den Flüssigkeitsspeicher 18 mit der kapillaren Struktur 40 verbinden. Die gleichmäßige Anordnung der Elemente der Verbindungseinrichtung 42 erlaubt eine isotrope Flüssigkeitsversorgung der kapillaren Struktur. Die Elemente der Verbindungseinrichtung 42 erstrecken sich in dieser Ausführungsform durch den Nebenluftkanal 5 und sind vorzugsweise gekapselt.

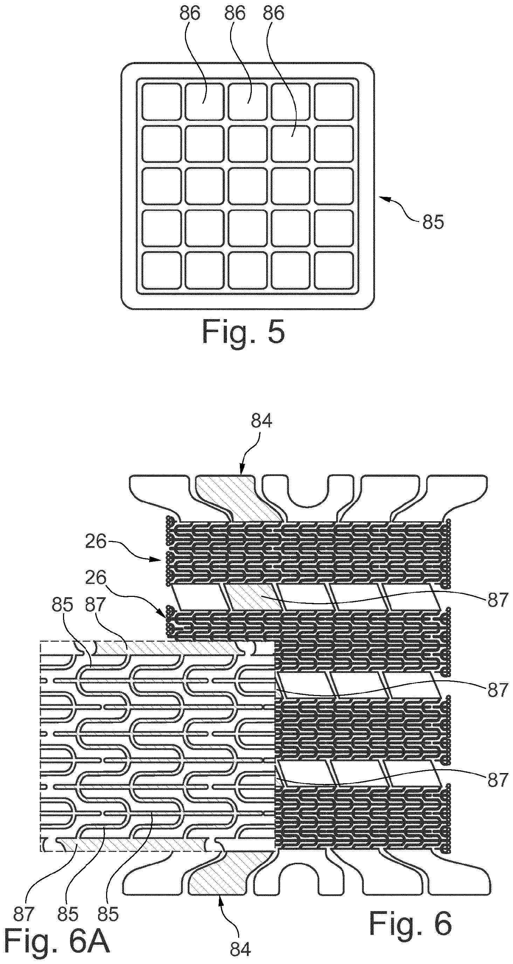

Alternative Ausführungen der Heizstruktur 26 sind in den Figuren 5 und 6 gezeigt. Deren Form mit Heizleitern 85 ist an bionische Strukturen, wie sie in Form von Mikrofibrillen insbesondere in Holzfaserzellen oder Tracheen zu finden sind, angelehnt, siehe Figur 5. Ebenso die kapillaren Struktur 40 mit den Strukturelementen 44 und/oder die Verbindungseinrichtung 42 können vorteilhaft in Ihrer Form an die Form von Mikrofibrillen angelehnt sein. Vorteilhafte Abmessungen der Kanäle 86 zwischen den Heizleitern 85, d.h. der freie Abstand zwischen benachbarten Heizstrukturen 85, liegen im Bereich zwischen 10 µm und 0.5 mm, weiter vorteilhaft zwischen 15 µm und 150 µm.

Eine bionische Heizstruktur 26 mit entsprechend geformten und beabstandeten Heizleitern 85, die durch Leitungsabschnitte 87 seriell miteinander verbunden sein können, ist in Figur 6 gezeigt. Die Figur 5A ist eine vergrößerte auszugsweise Darstellung der Figur 6 im Bereich einer Heizstruktur 26. Wie in den Figuren 6 und 6A erkennbar ist, ergibt sich durch die vorteilhafte netzartige Verknüpfung der Heizleiter 85 bzw. der Heizelementgeometrie eine dichte Flächenstruktur mit großer Flächenabdeckung, was eine besonders schnelle und effiziente Verdampfung von Flüssigkeit ermöglich. Elektroden 84 sind zum Anlegen der Heizspannung Uh an das Heizelement 36 mit diesem verbindbar.

## Patentansprüche

1. Verdampfereinheit (20) für einen Inhalator mit einem Heizelement (36), wobei die Verdampfereinheit (20) zum Verdampfen von Flüssigkeit eingerichtet ist, wobei die verdampfte Flüssigkeit von einem durch die Verdampfereinheit (20) strömenden Luftstrom aufgenommen wird, wobei die Verdampfereinheit (20) mindestens eine luftdurchlässige kapillare Struktur (40) mit einer Oberfläche (41) aufweist, die zur Vorwärmung der Flüssigkeit beheizbar sowie mindestens teilweise mit Flüssigkeit benetzbar ist und durch Kapillarwirkung mit Flüssigkeit versorgt wird, wobei das Heizelement (36) zum Erwärmen des durch die Verdampfereinheit (20) strömenden Luftstroms eingerichtet ist, wobei die kapillare Struktur (40) stromabwärts von dem Heizelement (36) angeordnet ist, so dass der erwärmte Luftstrom zur Verdampfung der vorgewärmten Flüssigkeit führt, **dadurch gekennzeichnet, dass** die Verdampfereinheit (20) einen Nebenluftkanal (5) aufweist, in dem in die Verdampfereinheit (20) eingesaugte Luft unter Umgehung des Heizelements (36) und des Verdampfers (22) strömt.

2. Verdampfereinheit (20) nach Anspruch 1, **dadurch gekennzeichnet, dass** die kapillare Struktur (40) aus einem elektrisch leitfähigen Material besteht.

3. Verdampfereinheit nach Anspruch 2, **dadurch gekennzeichnet, dass** das Heizelement (36) zum Aufheizen des Luftstroms auf eine Temperatur oberhalb einer Siedetemperatur des Flüssigkeitsgemisches eingerichtet ist und/oder dass die kapillare Struktur (40) zum Aufheizen des Liquids auf eine Temperatur unterhalb einer Siedetemperatur des Flüssigkeitsgemisches und/oder auf eine Temperatur von mindestens 100 °C, vorzugsweise mindestens 150 °C, weiter vorzugsweise mindestens 200 °C und noch weiter vorzugsweise mindestens 250 °C eingerichtet ist.

4. Verdampfereinheit nach einem der vorangehenden Ansprüche, **dadurch gekennzeichnet, dass** die kapillare Struktur (40) mittels einer ersten elektrischen/elektronischen Einheit (70) beheizbar ist, wobei die erste elektrische/elektronische Einheit (70) eingerichtet ist, das Heizelement (36) zu steuern und/oder zu regeln.

5. Verdampfereinheit nach einem der vorangehenden Ansprüche, **dadurch gekennzeichnet, dass** eine erste elektrische/elektronische Einheit (70) eingerichtet ist, das Heizelement (36) zu steuern und/oder zu regeln und die kapillare Struktur (40) mittels einer zweiten elektrischen/elektronischen Einheit (71) beheizbar ist.

6. Verdampfereinheit nach einem der vorangehenden Ansprüche, **dadurch gekennzeichnet, dass** die kapillare Struktur (40) vlies- oder gewebeartig, insbesondere in Form eines gefalteten oder gerollten Metallgewebes ist.

7. Verdampfereinheit nach einem der vorangehenden Ansprüche, **dadurch gekennzeichnet, dass** die kapillare Struktur (40) eine mikromechanisch hergestellte Struktur mit einem Substrat und in dem Substrat verlaufenden Kanälen und/oder Rippen ist.

8. Verdampfereinheit nach einem der vorangehenden Ansprüche, **dadurch gekennzeichnet, dass** sich Form und Geometrie der der Strukturelemente (44) der kapillaren Struktur (40) und/oder der Heizstrukturen (26) des Heizelements (36) an bionische Strukturen, ähnlich wie beispielsweise Mikrofibrillen (85), anlehnen.

9. Verdampfereinheit nach einem der vorherigen Ansprüche, **dadurch gekennzeichnet, dass** mindestens eine Betriebsbedingung, beispielsweise die Temperatur, der kapillaren Struktur (40) mit einem Sensor (7) messbar ist.

10. Verdampfereinheit nach einem der vorigen Ansprüche, **dadurch gekennzeichnet, dass** die kapillare Struktur (40) mittels einer Verbindungseinrichtung (42) mit einem Flüssigkeitsspeicher (18) verbindbar ist.

11. Verdampfereinheit nach Anspruch 10, **dadurch gekennzeichnet, dass** die Verbindungseinrichtung (42) dazu eingerichtet ist, Flüssigkeit mittels Kapillarwirkung zu fördern.

12. Verdampfereinheit nach Anspruch 10 oder 11, **dadurch gekennzeichnet, dass** die Verbindungseinrichtung (42) ein Vlies ist.

13. Verdampfereinheit nach einem der Ansprüche 10 bis 12, **dadurch gekennzeichnet, dass** die Verbindungseinrichtung (42) aus einem elektrisch isolierenden Material besteht.

14. Verdampfereinheit nach einem der Ansprüche 10 bis 13, **dadurch gekennzeichnet, dass** die Verbindungseinrichtung (42) gekapselt ist.

15. Verdampfereinheit nach einem der vorangehenden Ansprüche, umfassend einen Flüssigkeitsspeicher (18), **dadurch gekennzeichnet, dass** der Flüssigkeitsspeicher (18) ringförmig um den Verdampfer (22) angeordnet ist.

16. Verdampfereinheit nach einem der vorangehenden Ansprüche, **dadurch gekennzeichnet, dass** stromabwärts von dem Verdampfer (22) eine Mischungszone (25) vorgesehen ist, in der eine Vermischung eines insbesondere mit verdampfter Flüssigkeit angereicherten Hauptluftstroms (F2) mit einem Nebenluftstrom (F3) erfolgt.

17. Verdampfereinheit nach Anspruch 16, **dadurch gekennzeichnet, dass** das Verhältnis des Volumenstroms durch den Hauptluftstrom (F2) und den Nebenstrom (F3) einstellbar ist.

18. Verdampfereinheit (20) nach einem der vorangehenden Ansprüche, **dadurch gekennzeichnet, dass** das Heizelement (36) und der Verdampfer (22) in einem luftdurchströmten Hauptluftkanal (3) angeordnet sind.

19. Verdampfereinheit nach Anspruch 18, **dadurch gekennzeichnet, dass** der Hauptluftkanal (3) durch den Innenraum eines beidseitig offenen, vorzugsweise eintritts- und/oder austrittsseitig frei durchströmbaren Verdampferrohrs (2) gebildet ist.

20. Verdampfereinheit nach einem der vorangehenden Ansprüche, **dadurch gekennzeichnet, dass** der Nebenluftkanal (5) einen Eintritt (60) und einen Austritt (61) aufweist, wobei bevorzugt der Eintritt (60) vor dem Heizelement (36) und/oder vor dem Verdampfer (22) und/oder der Austritt (61) nach dem Heizelement (36) und/oder nach dem Verdampfer (22) angeordnet ist.

21. Verdampfereinheit nach Anspruch 18, **dadurch gekennzeichnet, dass** der Hauptluftkanal (3) innerhalb des Nebenluftkanals (5) angeordnet ist.

22. Verdampfereinheit (20) nach einem der vorangehenden Ansprüche, **dadurch gekennzeichnet, dass** die kapillare Struktur (40) metallfrei ist.

23. Kartusche (21) mit einem Flüssigkeitsspeicher (18) und einer Verdampfereinheit (20) nach einem der vorangehenden Ansprüche.

24. Inhalator (10) mit einer Verdampfereinheit (20) nach einem der Ansprüche 1 bis 22.

25. Verfahren zum Steuern einer Verdampfereinheit (20) nach einem der Ansprüche 1 bis 22, **dadurch gekennzeichnet, dass** die kapillare Struktur (40) und/oder das Heizelement (36) nicht-kontinuierlich, insbesondere gepulst, beheizt wird.

26. Verfahren nach Anspruch 25, **dadurch gekennzeichnet, dass** ein zeitlicher Heizspannungsverlauf Uhk(t) für die kapillare Struktur (40) so vorgegeben ist, dass ein Nachströmen von Flüssigkeit gewährleistet ist.

27. Verfahren nach einem der Ansprüche 25 bis 26, **dadurch gekennzeichnet, dass** die Heizleistung des Heizelements (36) und/oder der kapillaren Struktur (40) an einer Temperaturobergrenze abgeregelt wird.

28. Verfahren nach einem der Ansprüche 25 bis 27, **dadurch gekennzeichnet, dass** die Heizleistung des Heizelements (36) und/oder der kapillaren Struktur (40) so geregelt ist, dass eine geführte Verdampfung nach Temperaturvorgaben gewährleistet ist.

## Claims

1. Evaporator unit (20) for an inhaler comprising a heating element (36), the evaporator unit (20) being designed to evaporate liquid, the evaporated liquid being taken up by an airflow flowing through the evaporator unit (20), wherein the evaporator unit (20) comprises at least one air-permeable capillary structure (40) having a surface (41) that is heatable in order to preheat the liquid and can be wetted, at least in part, by liquid and is supplied with liquid by capillary action, the heating element (36) being designed to heat the airflow flowing through the evaporator unit (20), the capillary structure (40) being arranged downstream of the heating element (36) such that the heated airflow leads to evaporation of the preheated liquid, **characterised in that** the evaporator unit (20) comprises a secondary air channel (5) in which air sucked into the evaporator unit (20) flows whilst bypassing the heating element (36) and the evaporator (22).

2. Evaporator unit (20) according to claim 1, **characterised in that** the capillary structure (40) consists of an electrically conductive material.

3. Evaporator unit according to claim 2, **characterised in that** heating element (36) is designed to heat the airflow to a temperature above a boiling point of the liquid mixture, and/or **in that** the capillary structure (40) is designed to heat the liquid to a temperature below a boiling point of the liquid mixture and/or to a temperature of at least 100°C, preferably at least 150°C, more preferably at least 200°C and even more preferably at least 250°C.

4. Evaporator unit according to any of the preceding claims, **characterised in that** the capillary structure (40) is heatable by means of a first electrical/electronic unit (70), the first electrical/electronic unit (70) being designed to control the heating element (36).

5. Evaporator unit according to any of the preceding claims, **characterised in that** a first electrical/electronic unit (70) is designed to control the heating element (36), and the capillary structure (40) is heatable by means of a second electrical/electronic unit (71).

6. Evaporator unit according to any of the preceding claims, **characterised in that** the capillary structure (40) is non-woven or woven, in particular in the form of a folded or rolled woven metal fabric.

7. Evaporator unit according to any of the preceding claims, **characterised in that** the capillary structure (40) is a micromechanically produced structure comprising a substrate and channels and/or grooves running in the substrate.

8. Evaporator unit according to any of the preceding claims, **characterised in that** the form and geometry of the structural elements (44) of the capillary structure (40) and/or of the heating structures (26) of the heating element (36) follows bionic structures, similar to microfibrils (85) for example.

9. Evaporator unit according to any of the preceding claims, **characterised in that** at least one operating condition, for example the temperature, of the capillary structure (40) can be measured using a sensor (7).

10. Evaporator unit according to any of the preceding claims, **characterised in that** the capillary structure (40) can be connected to a liquid store (18) by a connection means (42).

11. Evaporator unit according to claim 10, **characterised in that** the connection means (42) is designed to feed liquid by means of capillary action.

12. Evaporator unit according to either claim 10 or claim 11, **characterised in that** the connection means (42) is a non-woven fabric.

13. Evaporator unit according to any of claims 10 to 12, **characterised in that** the connection means (42) consists of an electrically insulating material.

14. Evaporator unit according to any of claims 10 to 13, **characterised in that** the connection means (42) is encapsulated.

15. Evaporator unit according to any of the preceding claims including a liquid store (18), **characterised in that** the liquid store (18) is arranged annularly around the evaporator (22).

16. Evaporator unit according to any of the preceding claims, **characterised in that** a mixing zone (25) is provided downstream of the evaporator (22), in which zone a primary airflow (F2) in particular enriched with evaporated liquid is mixed with a secondary airflow (F3).

17. Evaporator unit according to claim 16, **characterised in that** the volumetric flow rate can be set by means of the primary airflow (F2) and the secondary airflow (F3).

18. Evaporator unit (20) according to any of the preceding claims, **characterised in that** the heating element (36) and the evaporator (22) are arranged in an air-permeable primary air channel (3).

19. Evaporator unit according to claim 18, **characterised in that** the primary air channel (3) is formed by the inner space of an evaporator tube (2) that is open at both ends, preferably an evaporator tube which allows free passage of a flow at the inlet and/or the outlet thereof.

20. Evaporator unit according to any of the preceding claims, **characterised in that** the secondary air channel (5) comprises an inlet (60) and an outlet (61), the inlet (60) preferably being arranged upstream of the heating element (36) and/or upstream of the evaporator (22) and/or the outlet (61) being arranged downstream of the heating element (36) and/or downstream of the evaporator (22).

21. Evaporator unit according to claim 18, **characterised in that** the primary air channel (3) is arranged inside the secondary air channel (5).

22. Evaporator unit (20) according to any of the preceding claims, **characterised in that** the capillary structure (40) is free of metal.

23. Cartridge (21) comprising a liquid store (18) and an evaporator unit (20) according to any of the preceding claims.

24. Inhaler (10) comprising an evaporator unit (20) according to any of the preceding claims.

25. Method for controlling an evaporator unit (20) according to any of claims 1 to 22, **characterised in that** the capillary structure (40) and/or the heating element (36) are heated in a non-continuous, in particular pulsed manner.

26. Method according to claim 25, **characterised in that** a temporal heating voltage profile Uhk(t) for the capillary structure (40) is specified such that a subsequent flow of liquid is ensured.

27. Method according to any of claims 25 to 26, **characterised in that** the heating power of the heating element (36) and/or of the capillary structure (40) is kept at an upper temperature limit.

28. Method according to any of claims 25 to 27, **characterised in that** the heating power of the heating element (36) and/or of the capillary structure (40) is controlled such that evaporation is ensured according to temperature sspecifications.

## Revendications

1. Unité évaporateur (20) pour un inhalateur avec un élément chauffant (36), l'unité évaporateur (20) étant configurée pour l'évaporation de liquide, le liquide évaporé étant absorbé par un flux d'air circulant à travers l'unité évaporateur (20), l'unité évaporateur (20) comportant au moins une structure capillaire (40) perméable à l'air avec une surface (41) qui peut être chauffée pour le préchauffage du liquide ainsi qu'humectée au moins partiellement avec du liquide et qui est alimentée en liquide par capillarité, l'élément chauffant (36) étant configuré pour chauffer le flux d'air circulant à travers l'unité évaporateur (20), la structure capillaire (40) étant disposée en aval de l'élément chauffant (36), de telle sorte que le flux d'air chauffé entraîne l'évaporation du liquide préchauffé, **caractérisée en ce que** l'unité évaporateur (20) comporte un conduit d'air secondaire (5), dans lequel de l'air aspiré dans l'unité évaporateur (20) circule en contournant l'élément chauffant (36) et l'évaporateur (22).

2. Unité évaporateur (20) selon la revendication 1, **caractérisée en ce que** la structure capillaire (40) est constituée d'un matériau électriquement conducteur.

3. Unité évaporateur selon la revendication 2, **caractérisée en ce que** l'élément chauffant (36) est configuré pour le chauffage du flux d'air à une température supérieure à une température d'ébullition du mélange de liquide et/ou **en ce que** la structure capillaire (40) est configurée pour le chauffage du liquide à une température inférieure à une température d'ébullition du mélange de liquide et/ou à une température d'au moins 100 °C, de préférence au moins 150 °C, plus préférentiellement au moins 200 °C et de manière encore plus préférentielle au moins 250 °C.

4. Unité évaporateur selon l'une des revendications précédentes, **caractérisée en ce que** la structure capillaire (40) peut être chauffée au moyen d'une première unité électrique/électronique (70), la première unité électrique/électronique (70) étant configurée pour commander et/ou réguler l'élément chauffant (36).

5. Unité évaporateur selon l'une des revendications précédentes, **caractérisée en ce qu'**une première unité électrique/électronique (70) est configurée pour commander et/ou réguler l'élément chauffant (36) et la structure capillaire (40) peut être chauffée au moyen d'une seconde unité électrique/électronique (71).

6. Unité évaporateur selon l'une des revendications précédentes, **caractérisée en ce que** la structure capillaire (40) est de type non tissé ou tissé, en particulier sous la forme d'un tissu métallique plié ou roulé.

7. Unité évaporateur selon l'une des revendications précédentes, **caractérisée en ce que** la structure capillaire (40) est une structure fabriquée de manière micromécanique avec un substrat et des conduits et/ou nervures s'étendant dans le substrat.

8. Unité évaporateur selon l'une des revendications précédentes, **caractérisée en ce que** la forme et la géométrie des éléments de structure (44) de la structure capillaire (40) et/ou des structures chauffantes (26) de l'élément chauffant (36) s'inspirent de structures bioniques, similaires par exemple à des microfibrilles (85).

9. Unité évaporateur selon l'une des revendications précédentes, **caractérisée en ce qu'**au moins une condition de fonctionnement, par exemple la température, de la structure capillaire (40) peut être mesurée avec un capteur (7).

10. Unité évaporateur selon l'une des revendications précédentes, **caractérisée en ce que** la structure capillaire (40) peut être reliée à un réservoir de liquide (18) au moyen d'un dispositif de liaison (42).

11. Unité évaporateur selon la revendication 10, **caractérisée en ce que** le dispositif de liaison (42) est configuré pour transporter du liquide par capillarité.

12. Unité évaporateur selon la revendication 10 ou 11, **caractérisée en ce que** le dispositif de liaison (42) est un nontissé.

13. Unité évaporateur selon l'une des revendications 10 à 12, **caractérisée en ce que** le dispositif de liaison (42) est constitué d'un matériau électriquement isolant.

14. Unité évaporateur selon l'une des revendications 10 à 13, **caractérisée en ce que** le dispositif de liaison (42) est encapsulé.

15. Unité évaporateur selon l'une des revendications précédentes, comprenant un réservoir de liquide (18), **caractérisée en ce que** le réservoir de liquide (18) est disposé dans une forme annulaire autour de l'évaporateur (22).

16. Unité évaporateur selon l'une des revendications précédentes, **caractérisée en ce qu'**une zone de mélange (25) est prévue en aval de l'évaporateur (22), dans laquelle un mélange d'un flux d'air principal (F2), en particulier enrichi de liquide évaporé, avec un flux d'air secondaire (F3) est effectué.

17. Unité évaporateur selon la revendication 16, **caractérisée en ce que** le rapport du débit volumétrique peut être réglé par le flux d'air principal (F2) et le flux d'air secondaire (F3).

18. Unité évaporateur (20) selon l'une des revendications précédentes, **caractérisée en ce que** l'élément chauffant (36) et l'évaporateur (22) sont disposés dans un conduit d'air principal (3) dans lequel circule de l'air.

19. Unité évaporateur selon la revendication 18, **caractérisée en ce que** le conduit d'air principal (3) est formé par l'espace intérieur d'un tube d'évaporateur (2) ouvert des deux côtés, de préférence susceptible d'être parcouru librement côté entrée et/ou côté sortie.

20. Unité évaporateur selon l'une des revendications précédentes,
**caractérisée en ce que** le conduit d'air secondaire (5) comporte une entrée (60) et une sortie (61), l'entrée (60) étant de préférence disposée avant l'élément chauffant (36) et/ou avant l'évaporateur (22) et/ou la sortie (61) étant de préférence disposée après l'élément chauffant (36) et/ou après l'évaporateur (22).

21. Unité évaporateur selon la revendication 18, **caractérisée en ce que** le conduit d'air principal (3) est disposé à l'intérieur du conduit d'air secondaire (5).

22. Unité évaporateur (20) selon l'une des revendications précédentes, **caractérisée en ce que** la structure capillaire (40) est sans métal.

23. Cartouche (21) avec un réservoir de liquide (18) et une unité évaporateur (20) selon l'une des revendications précédentes.

24. Inhalateur (10) avec une unité évaporateur (20) selon l'une des revendications 1 à 22.

25. Procédé de commande d'une unité évaporateur (20) selon l'une des revendications 1 à 22, **caractérisé en ce que** la structure capillaire (40) et/ou l'élément chauffant (36) sont chauffés de manière discontinue, en particulier de manière pulsée.

26. Procédé selon la revendication 25, **caractérisé en ce qu'**une variation temporelle de la tension de chauffage Uhk(t) pour la structure capillaire (40) est prédéfinie de telle manière qu'une continuation de l'écoulement de liquide est garantie.

27. Procédé selon l'une des revendications 25 à 26, **caractérisé en ce que** la puissance calorifique de l'élément chauffant (36) et/ou de la structure capillaire (40) est régulée avec précision sur une limite supérieure de température.

28. Procédé selon l'une des revendications 25 à 27, **caractérisé en ce que** la puissance calorifique de l'élément chauffant (36) et/ou de la structure capillaire (40) est régulée de telle manière qu'une évaporation guidée selon des consignes de température est garantie.
